(19) **Europäisches Patentamt**

**European Patent Office**

**Office européen des brevets**

(11) Veröffentlichungsnummer: **0 279 382 B1**

(12) **EUROPÄISCHE PATENTSCHRIFT**

(45) Veröffentlichungstag der Patentschrift: **07.08.91**

(51) Int. Cl.⁵: **A61K 35/78**

(21) Anmeldenummer: **88102106.7**

(22) Anmeldetag: **12.02.88**

(54) Warzenmittel.

(30) Priorität: **18.02.87 DE 3705151**

(43) Veröffentlichungstag der Anmeldung:
**24.08.88 Patentblatt 88/34**

(45) Bekanntmachung des Hinweises auf die
Patenterteilung:
**07.08.91 Patentblatt 91/32**

(84) Benannte Vertragsstaaten:
**AT BE CH DE ES FR GB GR IT LI LU NL SE**

(56) Entgegenhaltungen:
**EP-A- 0 087 861**
**FR-A- 2 342 071**
**FR-A- 2 504 009**

**G. MADAUS, "Lehrbuch der Biologischen
Heilmittel", Band II, Seiten
1319,1326,1327,1013,1014; Band III, Seiten
1907-1909,1938, Thieme Verlag, Leipzig, DE**

**G. GARNIER et al.: "Ressources médicinales
de la flore francaise", Band I, 1961, Seiten
590-592,828-831,422-424, Vigor Frères éditeurs, Paris, FR**

**Homöopathisches Arzneibuch, 1. Ausgabe,
Seiten V-IX und 20-28 (1978)**

(73) Patentinhaber: **Lünemann, Brigitte**
**Alter Fischmarkt 1**
**W-4400 Münster(DE)**

(72) Erfinder: **Lünemann, Brigitte**
**Alter Fischmarkt 1**
**W-4400 Münster(DE)**

(74) Vertreter: **Brehm, Hans-Peter, Dr. Dipl.-Chem.
et al
Patentanwälte Kern, Brehm & Partner Albert-
Rosshaupter-Strasse 73
W-8000 München 70(DE)**

**Beschreibung**

Die Erfindung betrifft ein Warzenmittel auf der Basis von Bestandteilen der Pflanze Euphorbia cyparissias. Die Wirksamkeit des Neuen Warzenmittels konnte sowohl am Menschen wie am Tier nachgewiesen werden.

Warzen werden durch Papillomviren übertragen. In der Zwischenzeit sind über vierzig Typen von Papillomviren charakterisiert worden, welche unterschiedliche Warzen hervorrufen können.

Die Entstehung von Warzen beruht in den meisten Fällen auf direktem Kontakt mit Viruspartikeln. Neben Menge und Virulenz der Viruspartikeln hängt die Gefahr einer Infektion auch von der Empfänglichkeit des Patienten und vom Zustand seines Hautmilieus ab. Es gibt Anzeichen dafür, daß Warzen bei Patienten mit geschwächter körpereigener Immunabwehr deutlich häufiger auftreten.

Typisch für die nicht-malignen Warzenerkrankungen ist die Neigung zur Spontanheilung. Auch hier werden die bislang noch nicht vollständig geklärten Ursachen in Mechanismen der körpereigenen Immunabwehr gesucht.

Bislang erfolgt die Warzenbehandlung - abgesehen von unmittelbaren Einwirkungen durch Vereisung, Entfernung mit dem scharfen Löffel oder mit der Diathermieschlinge sowie chirurgische Exzision - ausschließlich durch Aufbringung lokal wirkender Mittel. Bekannte, lokal anwendbare Warzenmittel enthalten Salicylsäure, gegebenenfalls in Kombination mit Milchsäure, Eisessig und/oder Fluorouracil; für die Anwendung in hartnäckigen Fällen können Warzenmittel auch hochzytotoxische Substanzen, wie Podophyllin enthalten. Dr. R.F. Weiß führt im "Lehrbuch der Phytotherapie" (6. Auflage, Hippokrates Verlag, Stuttgart, 1986, S. 414) aus, daß "Warzen heute als Virusinfekt aufgefaßt werden. "Gemeine Warzen" (verrucae vulgaris) lassen sich mit dem frischen Milchsaft der Zypressen-Wolfsmilch Euphorbia cyparissias entfernen. Der Saft der frischen Pflanze wird auf die Warze aufgetragen, muß dort eintrocknen und möglichst lange verweilen. Der Saft ist weiß und reich an hautreizenden Substanzen, wie dem Lacton, Euphorbon und Harzen. Es ist ungewiß, ob die Wirkung auf einem virushemmenden Effekt beruht".

Auch im "Lehrbuch der biologischen Heilmittel" von G. Madaus (Thieme Verlag, Leipzig 1938) wird darauf hingewiesen (Bd. II, S. 1319, 1326, 1327), "daß bei Warzen die äußerliche Anwendung des Saftes von Euphorbia cyparissias von Nutzen ist". Ferner wird erwähnt, "daß den Wolfsmilchgewächsen eine starke antitoxinbildende und immunstoffliefernde Wirkung innewohnen soll".

Ferner weist G. Madaus in obigem Werk (Band II, S. 1013 und 1014) darauf hin, "daß Flecken und Makel der Haut, wie Flechten und Zittermale durch äußerliche Verwendung von Clematis vertrieben werden sollen".

Weiterhin führt G. Madaus in obigem Werk aus (Band III, S. 1907-1909), "daß Seidelbast (Mezereum) in geringen Gaben sehr gut bei Dermatopathien wirken soll, die durch heftiges Jucken charakterisiert sind". Beispielsweise wird die Anwendung bei Ekzemen des Handrückens empfohlen.

Die Praxis zeigt, daß diese bekannten, lokal applizierbaren Warzenmittel nicht immer zum Erfolg führen. Teilweise kehren die Warzen nach Absetzung der Therapie zurück, auch nach mehrmaliger operativer Entfernung können im Narbengebiet erneut Warzen auftreten. Gerade auch für solche hartnäckigen Fälle erscheint es wünschenswert, ein Warzenmittel bereitzustellen, das über einen anderen Wirkungsmechanismus eine Behandlung von Warzen ermöglicht.

Davon ausgehend besteht die Aufgabe der vorliegenden Erfindung darin, ein neues Warzenmittel bereitzustellen.

Ausgehend von einem Warzenmittel auf der Basis von Bestandteilen der Pflanze Euphorbia cyparissias ist die erfindungsgemäße Lösung dieser Aufgabe gekennzeichnet durch ein peroral verabreichbares Gemisch in Form von Urtinkturen mit einem Gehalt an

(a) 0,5 bis 2 Gew.-Teile Euphorbia cyparissias, Verdünnungsgrad D3 bis D6;

(b) 0,5 bis 2 Gew.-Teile Clematis recta, Verdünnungsgrad D2 bis D4 und

(c) 0,5 bis 2 Gew.-Teile Mezereum, Verdünnungsgrad D2 bis D4.

Das erfindungsgemäße Warzenmittel wird peroral verabreicht und bewirkt eine Behandlung von durch Papillomviren hervorgerufener Warzen bei Mensch und Tier über eine Stimulierung der körpereigenen Immunabwehr.

Für eine Anzahl homöopatischer Phytopräparate ist in der Zwischenzeit nachgewiesen worden, daß sie eine unspezifische Stimulierung des Immunsystems hervorrufen können.

Dies gilt sowohl für die zellulären wie für die humoralen Abwehrsysteme; vgl. insbesondere den Beitrag von Prof. Dr. H. Wagner, München, zu "Immunstimulantien und Phytotherapeutika" in Zeitschrift für Phytotherapie 7, S. 91 bis 98 (1986). Zumindest ein Teil der dort angegebenen immunstimulierenden Phytopräparate (vgl. Tab. I) ist auch als Agens oder Adjuvans des erfindungsgemäßen Warzenmittels brauchbar. Allerdings wird dort die Behandlung von Warzen über ein peroral verabreichbares, das Immunsystem stimulierendes Mittel nicht angesprochen. Auch aus der Volksheilkunde ist die perorale Behandlung von Warzen bislang nicht bekannt.

Das Präparat wurde zumeist dreimal täglich in einer Dosierung von etwa 10 bis 20 Tropfen verabreicht. Die Einnahmedauer schwankte zwischen zehn Tagen und vier Wochen. In einer mehrjährigen Praxis wurde eine signifikante Wirkung bei Patienten mit vulgären Warzen beobachtet, die typischerweise auf humane Papillomviren (HPV) vom Typ 1, 2,3,4 und 7 zurückzuführen sind. In vielen Fällen waren die Patienten vorher mehrfach erfolglos konservativ therapiert worden. Nach Verabreichung des erfindungsgemäßen Warzenmittels im genannten Zeitraum fielen die papillomatösen Hyperkeratosen ohne Narbenbildung ab. Dornwarzen der Fußsohlen und dergleichen bildeten sich bis zum Verschwinden zurück.

Mit dem erfindungsgemäßen Präparat wird erstmalig ein Pharmakon bereitgestellt, das nicht nur eine unspezifische Paraimmunität induziert, sondern das eine gezielte Abwehrreaktion gegen Papillomviren provoziert. Das erfindungsgemäße Präparat vermag Immundefekte und Immunschwächen zu kompensieren, was besonders bedeutsam ist, weil praktisch keine einfachen und preiswerten Virustatika, insbesondere gegen Papillomviren, zur Verfügung stehen. Hier ist auch an den Veterinärbereich zu denken; beispielsweise konnten mit dem erfindungsgemäßen Warzenmittel hartnäckige Warzen bei Pferden beseitigt werden. Dank der extrem niedrigen Dosierung sind unerwünschte Nebenwirkungen bei Verabreichung des erfindungsgemäßen Warzenmittels ausgeschlossen.

Das erfindungsgemäße Warzenmittel soll wenigstens die oben angegebenen Komponenten (a), (b) und (c) enthalten. Damit kann auf den spezifischen Immundefekt eingewirkt werden, der - unter den Bedingungen der Vireninfektion - offensichtlich zur Entstehung der Warzen geführt hat. Darüber hinaus kann das erfindungsgemäße Warzenmittel zusätzlich eine oder mehrere Wahlkomponente(n) enthalten. Nach den bislang vorliegenden Erfahrungen verstärkt und/oder fördert ein Zusatz dieser Wahlkomponenten die Wirkung des erfindungsgemäßen Warzenmittels. Insbesondere kann mit diesen Wahlkomponenten auf eine allgemeine Immunsuppression positiv eingewirkt werden, wie sie gerade bei Erwachsenen häufig vorkommt. Zu diesen Wahlkomponenten gehören insbesondere:

(d) 0,5 bis 2 Gew.Teile Ranunculus, Verdünnungsgrad D2 bis D4;

(e) 0,5 bis 2 Gew.-Teile Veratrum album, Verdünnungsgrad D3 bis D6;

(f) 0,5 bis 2 Gew.-Teile Aconitum, Verdünnungsgrad D3 bis D6;

(g) 0,5 bis 2 Gew.-Teile Arnica, Verdünnungsgrad D2 bis D4.

Ein im Rahmen der vorliegenden Erfindung mit großem Erfolg eingesetztes, oral applizierbares Warzenmittel besteht beispielsweise aus

(a) 0,5 bis 2 Gew.-Teile Euphorbia cyparissias,

Verdünnungsgrad D3 bis D6;

(b) 0,5 bis 2 Gew.-Teile Clematis, Verdünnungsgrad D2 bis D4;

(c) 0,5 bis 2 Gew.-Teile Mezereum, Verdünnungsgrad D2 bis D4;

(d) 0,5 bis 2 Gew.-Teile Ranunculus, Verdünnungsgrad D2 bis D4;

(e) 0,5 bis 2 Gew.-Teile Veratrum Album, Verdünnungsgrad D3 bis D6;

(f) 0,5 bis 2 Gew.-Teile Aconitum, Verdünnungsgrad D3 bis D6, und

(g) 0,5 bis 2 Gew.-Teile Arnica, Verdünnungsgrad D2 bis D4.

In weiterer Ausbildung der vorliegenden Erfindung ist festgestellt worden, daß bestimmte weitere Zusätze in vielen Fällen eine günstige Wirkung haben, und die Wirksamkeit des erfindungsgemäßen Warzenmittels noch steigern können. Zu diesen weiteren Zusätzen gehören:

(h) 0,5 bis 2 Gew.-Teile Atropa belladonna, Verdünnungsgrad D3 bis D6;

(i) 0,5 bis 2 Gew.-Teile Cinchona succiruba, Verdünnungsgrad D2 bis D4;

(k) 0,5 bis 2 Gew.-Teile Eucalyptus globulus, Verdünnungsgrad D2 bis D4;

(l) 0,5 bis 2 Gew.-Teile Lycopodium, Verdünnungsgrad D3 bis D6;

(m) 0,5 bis 2 Gew.-Teile Nux vomica, Verdünnungsgrad D3 bis D6, und

0,5 bis 2 Gew.-Teile Sulfur, Verdünnungsgrad D4 bis D6.

Ein weiterer Gesichtspunkt der Erfindung betrifft - auch im Sinne einer zweiten Indikation - die Verwendung bekannter Phytopräparate mit immunstimulierender Wirkung, insbesondere und vorzugsweise die in den Patentansprüchen angegebenen Arzneimittelgemische zur Herstellung eines Arzneimittels, zur Behandlung von durch Papillomviren hervorgerufener Warzen an Mensch und Tier. Vorzugsweise ist hierbei das Warzenmittel für die perorale Anwendung konfektioniert. Die überraschende Wirkung bei dieser Verwendung, nämlich eine gezielte Provozierung körpereigener Abwehrmaßnahmen gegen die von Papillomviren induzierten Warzen beruht offensichtlich auf einem synergistischen Effekt, denn die Wirkung von Kombinationspräparaten ist wesentlich größer als diejenige der Einzelkomponenten. Besonders gute Wirkungen werden mit Kombinationspräparaten erzielt, wie sie in den Patentansprüchen angegeben sind.

Sämtliche Bestandteile des erfindungsgemäßen Warzenmittels stellen bekannte, homöopatische oder andere Arzneimittel dar, deren Herstellung und Potenzierung zum gewünschten Verdünnungsgrad in der Fachliteratur beschrieben ist. Soweit hier bekannt, sind sowohl die einzelnen Arzneimittel wie deren Gemische bislang nicht zur Aktivierung der körpereigenen Immunabwehr gegen Papillomviren eingesetzt worden, um ein gegen Warzen wirksames Arzneimittel bereitzustellen. Obwohl die genannten Arzneimittel bekannt sind, wird zur Gewährleistung einer vollständigen, in sich geschlossenen Offenbarung nachstehend deren Herstellung und Verdünnung kurz angegeben, wobei insbesondere auf das "Homöopatische Arzneibuch" in der Gesamtausgabe nach der Neufassung 1985 zurückgegriffen wird. Das "Homöopatische Arzneibuch" (HAB) ist im "Deutscher Apotheker-Verlag, Stuttgart, Govi-Verlag GmbH", Frankfurt, erschienen.

Soweit nicht anders angegeben, erfolgt die Herstellung der Urtinkturen und der flüssigen Dezimalverdünnungen entsprechend nachstehender, allgemeiner Vorschrift:

Die Pflanze oder die Pflanzenteile werden fein zerkleinert. Von einer Probe wird der Trocknungsverlust bestimmt. Die zerkleinerte Pflanzenmasse wird sofort mit mindestens der Hälfte ihres Gewichts Ethanol 86% versetzt und bei einer 20° C nicht übersteigenden Temperatur in gut verschlossenen Gefäßen gelagert.

Nach nachstehender Formel

$$A_3 = \frac{2 \times M \times T}{100} \; (kg)$$

wobei

M   für das Gewicht der Pflanzenmasse in Kilogramm und

T   für den Trocknungsverlust der Probe in Prozent steht,

wird die für die Pflanzenmasse erforderliche Menge Ethanol 86% ($A_3$) errechnet, die bereits zugesetzte Menge Ethanol davon abgezogen und der Rest mit dem Ansatz gemischt. Der Ansatz bleibt mindestens zehn Tage bei einer 20° C nicht übersteigenden Temperatur unter wiederholtem Umschütteln stehen. Danach wird abgepreßt und filtriert.

Die Einstellung auf eine bestimmte, gewünschte Dezimalverdünnung (Potenzierung) erfolgt nach folgender Vorschrift:

Die 1. Dezimalverdünnung (D1) wird aus
    3 Teilen Urtinktur und
    7 Teilen Ethanol 62%
hergestellt.

Die 2. Dezimalverdünnung (D2) wird aus
    1 Teil der 1. Dezimalverdünnung und
    9 Teilen Ethanol 62%
hergestellt.

Die 3. Dezimalverdünnung (D3) wird aus
    1 Teil der 2. Dezimalverdünnung und
    9 Teilen Ethanol 62%
hergestellt.

Die 4. Dezimalverdünnung (D4) wird aus
    1 Teil der 3. Dezimalverdünnung und
    9 Teilen Ethanol 43%
hergestellt.

Die Herstellung noch höherer Dezimalverdünnungen erfolgt in analoger Weise mit Ethanol 43%.

Entsprechend der vorstehenden Vorschrift oder einer im Einzelfall abgewandelten Vorschrift können die nachstehend angegebenen, sämtlich bekannten, flüssigen Arzneimittel-Verdünnungen erhalten werden.

(a) Euphorbia cyparissias, D4

Zur Herstellung der Urtinktur wird die ganze, frische blühende Pflanze von Euphorbia cyparissias L. verwendet. Die Urtinktur bildet eine braungrüne bis braungelbe Flüssigkeit mit indifferentem Geruch und scharfem bis bitterem Geschmack. Die relative Dichte der Urtinktur beträgt 0,895 bis 0,915 g/cm³. Der Trockenrückstand beträgt mindestens 1,6 und höchstens 3,4%.

(b) Clematis recta, D2

Zur Herstellung der Urtinktur werden die frischen, oberirdischen Teile blühender Pflanzen von Clematis recta L. verwendet. Die Urtinktur bildet eine braungrüne Flüssigkeit ohne besonderen Geruch und Geschmack. Ihre relative Dichte beträgt 0,0900 bis 0,915 g/cm³. Der Trockenrückstand muß mindestens 2,4% betragen.

(c) Mezereum, D3

Zur Herstellung der Urtinktur wird frische, vor Beginn der Blüte gesammelte Zweigrinde von Seidelbast (Kellerhals) verwendet. Die Dichte der Urtinktur beträgt 0,895 bis 0,910 g/cm³. Ihr Trockenrückstand beträgt 2 bis 4%.

(d) Ranunculus bulbosus, D3

Zur Herstellung der Urtinktur wird die ganze, frische, blühende Pflanze von Ranunculus bulbosus L. verwendet. Die Urtinktur bildet eine grünlichgelbe bis gelbbraune Flüssigkeit ohne besonderen Geruch. Sie weist eine relative Dichte von 0,900 bis 0,915 g/cm³ auf. Der Trockenrückstand beträgt mindestens 1,5 und höchstens 3,0%.

(e) Veratrum album, D4

Die Herstellung der Urtinktur erfolgt aus Pflanzenteilen von Veratrum album. Die Dichte der Urtinktur beträgt 0,895 bis 0,905 g/cm³. Der Trockenrückstand beträgt 1,8 bis 3,4%. Der Trockenrückstand selbst weist einen Mindestgehalt an Alkaloiden von 0,07% auf. Zu diesen Alkaloiden gehören insbesondere Veratrin und Gemerin.

**(f) Aconitum napellus, D4**

Zur Herstellung der Urtinktur werden die frischen, zu Beginn der Blütezeit gesammelten oberirdischen Teile und Wurzelknollen von Aconitum napellus L. verwendet. Abweichend zur oben angegebenen Vorschrift wird die Pflanzenmasse mit Ethanol 86% ausgelaugt, dessen Menge ($A_2$) nach folgender Formel berechnet wird:

$$A_2 = \frac{M \times T}{100} \ (kg)$$

wobei

M   für das Gewicht der Pflanzenmasse in Kilogramm und

T   für den Trocknungsverlust der Probe in Prozent steht.

Die Urtinktur bildet eine grünlich-gelbe, später bräunlich-gelbe Flüssigkeit von charakteristischem Geruch. Die relative Dichte beträgt 0,930 bis 0,942 $g/cm^3$. Der Trockenrückstand muß mindestens 2,0% betragen. Abweichend zur oben genannten, allgemeinen Potenzierungsvorschrift wird die 1. Dezimalverdünnung aus 2 Teilen Urtinktur und 8 Teilen Ethanol 30% hergestellt. Zur Herstellung der 2. Dezimalverdünnung (und in analoger Weise von D3 und D4) wird ein Teil der 1. Dezimalverdünnung mit 9 Teilen Ethanol 15% versetzt.

**(g) Arnica planta tota, D4**

Zur Herstellung der Urtinktur wird die ganze, frische blühende Pflanze von Arnica montana L. verwendet. Abweichend zur oben genannten, allgemeinen Vorschrift dient zur Herstellung der Urtinktur Ethanol 43%. Die Urtinktur bildet eine gelbe Flüssigkeit mit charakteristischem Geruch und bitterem Geschmack. Ihre relative Dichte beträgt 0,95 bis 0,969 $g/cm^3$. Der Trockenrückstand beträgt mindestens 1%. Die Herstellung der Dezimalverdünnungen erfolgt wie oben zu (f) Aconitum napellus angegeben.

**(h) Atropa belladonna, D4**

Zur Herstellung der Urtinktur wird die am Ende der Blütezeit gesammelte, ganze, frische Pflanze von Atropa belladonna L. ohne die verholzten unteren Stengelteile verwendet. Die Herstellung der Urtinktur erfolgt in gleicher Weise wie oben für Aconitum napellus angegeben. Die Urtinktur bildet eine braune Flüssigkeit von eigenartigem Geruch.

Ihre relative Dichte beträgt 0,932 bis 0,947 $g/cm^3$. Der Trockenrückstand beträgt mindestens 1,4%. Zu den wesentlichen Inhaltsstoffen gehört Atropin.

Die Herstellung der flüssigen Verdünnungen erfolgt wie oben für Aconitum napellus angegeben.

**(i) Cinchona succirubra (China), D2**

Zur Herstellung der Urtinktur werden die getrocknete Rinde jüngerer Stämme und ältere Zweige von Cinchona succirubra PAVON sowie deren Varietäten oder Hybriden verwendet. Die Urtinktur bildet eine rotbraune Flüssigkeit von angenehm bitterem Geschmack und enthält mindestens 0,45 und höchstens 0,50% Gesamtalkaloide, von denen mindestens 30 und höchstens 60% aus Alkoloiden vom Typ des Chinins bestehen. Abweichend zur oben angegebenen Vorschrift erfolgt die Herstellung der Urtinktur aus 1 Teil grob gepulverter Droge mit 10 Teilen Ethanol 62%. Die relative Dichte der Urtinktur beträgt 0,895 bis 0,908 $g/cm^3$. Der Trockenrückstand muß mindestens 2,4% betragen.

**(k) Eucalyptus globulus, D2**

Zur Herstellung der Urtinktur werden die getrockneten Blätter von Eucalyptus globulus LABILL. verwendet, die mindestens 1,5% ätherisches Öl enthalten. Die Herstellung der Urtinktur erfolgt wie oben für Cinchona succirubra (China) angegeben mit Ethanol 86%. Die Urtinktur bildet eine gelb- bis braungrüne Flüssigkeit mit dumpfwürzigem Geruch und schwachbitterem, arteigenem Geschmack. Ihre relative Dichte beträgt 0,833 bis 0,848 $g/cm^3$. Der Trockenrückstand muß mindestens 2,0% betragen.

**(l) Lycopodium, D4**

Zur Herstellung der Urtinktur werden zerriebene Sporen der Beerlappart Lycopodium clavatum lycopodiacea verwendet. Die Sporen enthalten ein fettes Öl und Spuren von Alkaloiden. Die relative Dichte der Urtinktur beträgt 0,83 bis 0,84 $g/cm^3$. Der Trockenrückstand beträgt 1,7 bis 2,4%.

**(m) Nux vomica, D4**

Zur Herstellung der Urtinktur werden die getrockneten Samen von Strychnos Nux vomica verwendet. Zu den Inhaltsstoffen gehören insbesondere die Alkaloide Strychnin und Prucin. Der Gesamtalkaloidgehalt beträgt 0,246 bis 0,255%. Die relative Dichte der Urtinktur beträgt 0,896 bis 0,901 $g/cm^3$. Der Trockenzustand macht 1,23 bis 1,68% aus.

**Sulfur, D6**

Zur Herstellung der Lösung (D4) wird ein Teil Schwefel mit 10.000 Teilen Ethanol 86% unter Rückflußkühlung 1 h lang gekocht. Die 5. Dezimalverdünnung wird mit Ethanol 86% und die 6. Dezimalverdünnung wird mit Ethanol 62% bereitet. Die relative Dichte der Lösung D4 beträgt 0,828 bis 0,833 g/cm³. Diese Lösung muß mindestens 0,009 und darf höchstens 0,011% Schwefel enthalten.

Beispiel 1:

Zur Herstellung eines erfindungsgemäßen Präparates werden gleiche Gewichtsmengen Euphorbia cyparissias D4, Clematis recta D2 und Mezereum D3 miteinander vermischt. Das fertige Präparat bildet eine klare, leicht gelbliche Flüssigkeit mit typischem Ethanolgeruch. Das Präparat wird in dieser Form (pur) in Form von Tropfen verabreicht. Eine typische Dosierung im Humanbereich sieht zwei- bis dreimal täglich die Verabreichung von 10 bis 15 Tropfen vor. Nach der Einnahme wird empfohlen, einen Schluck Wasser nachzutrinken, um den leicht brennenden Ethanolgeschmack zu beseitigen.

Die nachstehenden Fallbeispiele - aus einer Vielzahl ähnlicher Fallbeispiele ausgewählt - betreffen die erfolgreiche Anwendung des Warzenmittels nach Beispiel 1 an Kindern und Jugendlichen (bis ca. 16 Jahre). Bei ansonsten gesunden Kindern und Jugendlichen kann davon ausgegangen werden, daß das Immunsystem, d.h. die im wesentlichen verantwortliche T-Zellenreaktion und die B-Lymphozytenproduktion, ausreichend aktiv sind. Jedoch kann die spezifische Antwort auf die eingedrungenen Papillomviren Schwierigkeiten bereiten. Nach mehrjähriger Erfahrung ist hier die gezielte Stimulierung mit dem Warzenmittel nach Beispiel 1 völlig ausreichend und führt nach kurzer Zeit zur Abheilung.

Fall 1:

An einem sechsjährigen Mädchen waren beide Beine mit Warzen übersät. Dem Mädchen wurden dreimal täglich 10 Tropfen des Warzenmittels nach Beispiel 1 peroral verabreicht. Nach zehn Tagen war eine vollständige Abheilung festzustellen Auch nach Absetzung des Mittels traten keine neuen Warzen auf.

Fall 2:

Ein siebenjähriger Junge litt an Fußsohlenwarzen; ferner breiteten sich Warzenbeete über beide Kniee aus. Der Junge erhielt dreimal täglich 15 Tropfen des Warzenmittels nach Beispiel 1. Nach drei Wochen war eine vollständige, dauerhafte Abheilung festzustellen.

Fall 3:

Bei einem achtjährigen Mädchen breiteten sich über beide Handrücken Warzen aus. Es wurden dreimal täglich 15 Tropfen des Warzenmittels nach Beispiel 1 verabreicht. Nach sechs Tagen war eine praktisch vollständige Abheilung festzustellen.

Beispiel 2:

Zur Herstellung eines erfindungsgemäßen Warzenmittels werden miteinander vermischt:
8,3 g Euphorbia cyparissias, D4
8.3 g Clematis recta, D2
8,3 g Mezereum, D3
8,3 g Ranunculus, D3
7,2 g Veratrum album, D4
7,2 g Aconitum, D4, und
7,2 g Arnica, D3.

Dieses Warzenmittel wird in gleicher Weise eingesetzt und dosiert wie das Warzenmittel nach Beispiel 1.

Ersichtlich enthält das Warzenmittel nach Beispiel 2 zusätzlich zu den notwendigen Bestandteilen - Euphorbia cyparissias, Clematis recta und Mezereum - als Wahlkomponenten vier weitere Bestandteile, welche ebenfalls eine immunstimulierende Wirkung besitzen. Die Zusammensetzung nach Beispiel 2 ist insbesondere für Erwachsene vorgesehen. Weil mit zunehmendem Alter die Abwehrfähigkeit des Organismus generell abnimmt, muß das Zusammenwirken vieler immunstimulierender Substanzen ausgenutzt werden, um eine verbesserte Gesamtkonstitution zu erreichen, von der aus gezielt die Abwehr gegen Papillomviren erfolgen kann.

Die nachstehenden Fallbeispiele - aus einer Vielzahl ähnlicher Fallbeispiele ausgewählt - betreffen die erfolgreiche Anwendung des Warzenmittels nach Beispiel 2.

Fall 4:

Eine, als Arzthelferin tätige, 25-jährige Frau hatte hartnäckige Warzen am Handrücken, die mit der herkömmlichen, lokalen Therapie (Auftupfen von Salicylsäure-Lösung) nicht zu beheben waren. Die Frau nahm dreimal täglich 15 Tropfen des Warzenmittels nach Beispiel 2 ein. Nach ca. 20 Tagen war der Handrücken glatt. Auch nach Absetzung des Mittels kehrten die Warzen nicht zurück.

Fall 5:

Eine 29-jährige Frau litt seit langem an hartnäckigen Nagelbettwarzen. Es wurden dreimal täglich 15 Tropfen des Warzenmittels nach Beispiel 2 verabreicht. Nach drei Wochen waren die Nagelbettwarzen abgeheilt.

Fall 6:

Ein 30-jähriger Mann hatte sich eine einzelne, schmerzhafte Dornwarze am Daumen und eine weitere Nagelbettwarze zugezogen, die ihn bei seiner beruflichen Tätigkeit als Mechaniker erheblich behinderten. Es wurden dreimal täglich 15 Tropfen des Warzenmittels nach Beispiel 2 verabreicht. Bereits nach drei Tagen war eine positive Reaktion zu erkennen; die Warzen bildeten sich zurück. Nach zehn Tagen war eine vollständige, dauerhafte Heilung eingetreten.

Beispiel 3:

Zur Herstellung eines erfindungsgemäßen Warzenmittels wurden miteinander vermischt:
8,3 g Euphorbia cyparissias, D4
8,3 g Clematis recta, D2
8,3 g Mezereum, D3
8,3 g Ranunculus, D3
8,1 g Veratrum album, D4
7,2 g Aconitum, D4
7,2 g Arnica, D3
7,2 g Atropa belladonna, D4
7,2 g Cinchona succirubra (China), D2
7,2 g Eucalyptus, D2
8,3 g Lycopodium, D4
7,2 g Nux vomica, D4 und
8,3 g Sulfur, D6.

Der Gesamt-Ethanol-Gehalt des Präparates beträgt 67%. Dieses Warzenmittel wird analog zum Warzenmittel nach Beispiel 1 eingesetzt und dosiert. Es enthält weitere immunstimulierende Zusätze. Mit "Sulfur" is zusätzlich ein unspezifischer Reizkörper vorhanden, welcher die Zelloxidation steigern und eine Störung bestimmter Fermentfunktionen aufheben kann. Dieses Mittel ist vorzugsweise für besonders hartnäckige Fälle und für die Anwendung im Veterinärbereich vorgesehen.

Die nachstehenden Fallbeispiele - aus einer Vielzahl ähnlicher Fallbeispiele ausgewählt - betreffen die erfolgreiche Anwendung des Warzenmittels nach Beispiel 3.

Fall 7:

Eine 30-jährige Frau litt an einer papillomatösen Fingerwarze, die bereits erfolglos konventionell therapiert worden war (Salicylsäure-Präparate und Fluorouracil). Dieser Frau wurden dreimal täglich 15 Tropfen des Warzenmittels nach Beispiel 3 verabreicht. Nach 14 Tagen konnte eine Abheilung der Fingerwarze festgestellt werden.

Fall 8:

Eine 29-jährige Frau litt an Nagelbettwarzen, welche an beiden Daumen und den Zeigefingern bereits den gesamten Nagelfalz umschlossen haben. Zusätzlich waren bereits schwere, furchige Verformungen der Nägel aufgetreten. Nach einem ersten erfolglosen Operationsversuch hat die Patientin wegen der großen Schmerzhaftigkeit und Mißerfolgs jeden weiteren Operationsversuch abgelehnt. Der Frau wurde mit kurzdauernden Unterbrechungen zwei Monate lang das Warzenmittel nach Beispiel 3 in einer Dosierung von zweimal täglich 10 Tropfen verabreicht. Daraufhin konnte eine Abheilung der Warzen festgestellt werden.

Fall 9:

Bei einer 75-jährigen Frau waren beide Hände und die Unterarme mit Plaques überzogen. Seit Jahren bestand Verdacht auf Epidermodysplasie. Die Beweglichkeit der Finger war durch großflächige Keratosen stark eingeschränkt. Ein Versuch zur operativen Entfernung mißlang. Nachdem der Frau sechs Monate lang das erfindungsgemäße Warzenmittel nach Beispiel 3 verabreicht worden war (Dosis: zweimal täglich 10 Tropfen) konnte ein nahezu restloses Abheilen festgestellt werden.

Fall 10:

Eine 70-jährige Frau litt an einer papillomatösen Warze am Ringfinger. Nach zweimaliger Operation bildete sich erneut eine starke Warze im Narbengebiet. Nachdem der Frau zwei Monate lang das erfindungsgemäße Warzenmittel nach Beispiel 3 verabreicht worden war (Dosis: zweimal täglich 10 Tropfen) konnte eine Abheilung festgestellt werden.

Fall 11:

Ein 25-jähriger Mann litt an einer doppelt-erbsengroßen Warze in der Handfläche. Nachdem 14 Tage lang das erfindungsgemäße Warzenmittel nach Beispiel 3 verabreicht worden war (Dosis: zweimal täglich 10 Tropfen) ist die Warze verschwunden.

Fall 12:

Bei einem 35-jährigen Mann traten am gesamten Körper zahlreiche, sehr kleine Warzen auf. Eine unabhängig durchgeführte Bestimmung wichtiger Immunparameter bestätigte einen geschwächten Immunstatus. Der Patient erhielt dreimal täglich 15 Tropfen des Warzenmittels nach Beispiel 3. Nach drei Monaten waren auch die letzten Warzen verschwunden. Der Therapie-Erfolg war vollständig und dauerhaft.

Fall 13:

An einem 8-jährigen Wallach traten an beiden Vorderbeinen im Bereich zwischen Kron- und Fesselgelenk aufplatzende, massiv sekundärinfizierte Warzen mit einem Durchmesser von ca. 4 cm auf. Dem Tier wurden zusammen mit dem Hafer zweimal täglich 5 ml des erfindungsgemäßen Warzenmittels nach Beispiel 3 verabreicht. Als erste Reaktion war bereits nach wenigen Tagen ein Abheilen vom Rand her sichtbar. Nach vier Wochen war bis auf eine 0,5 cm² große, glatte gesunde Hautfläche auch die Behaarung wieder hergestellt.

**Patentansprüche**

1. Ein Warzenmittel
   auf der Basis von Bestandteilen der Pflanze Euphorbia cyparissias,
   gekennzeichnet durch
   ein peroral verabreichbares Gemisch in Form von Urtinkturen mit einem Gehalt an
   (a) 0,5 bis 2 Gew.-Teile Euphorbia cyparissias,
   Verdünnungsgrad D3 bis D6;
   (b) 0,5 bis 2 Gew.-Teile Clematis recta,
   Verdünnungsgrad D2 bis D4 und
   (c) 0,5 bis 2 Gew.-Teile Mezereum,
   Verdünnungsgrad D2 bis D4.

2. Das Warzenmittel nach Anspruch 1,
   dadurch gekennzeichnet, daß
   das Gemisch zusätzlich eine Urtinktur von Sulfur im Verdünnungsgrad D2 bis D6 enthält.

3. Das Warzenmittel nach Anspruch 1 oder 2,
   dadurch gekennzeichnet, daß
   das Gemisch zusätzlich
   (d) 0,5 bis 2 Gew.-Teile Ranunculus,
   Verdünnungsgrad D2 bis D4
   und/oder zusätzlich
   (e) 0,5 bis 2 Gew.-Teile Veratrum album,
   Verdünnungsgrad D3 bis D6
   und/oder zusätzlich
   (f) 0,5 bis 2 Gew.-Teile Aconitum,
   Verdünnungsgrad D3 bis D6
   und/oder zusätzlich
   (g) 0,5 bis 2 Gew.-Teile Arnica,
   Verdünnungsgrad D2 bis D4
   enthält.

4. Das Warzenmittel nach Anspruch 1 oder 2,
   gekennzeichnet durch
   ein peroral verabreichbares Präparat, enthaltend
   (a) 0,5 bis 2 Gew.-Teile Euphorbia cyparissias,
   Verdünnungsgrad D3 bis D6;

   (b) 0,5 bis 2 Gew.-Teile Clematis recta,
   Verdünnungsgrad D2 bis D4;
   (c) 0,5 bis 2 Gew.-Teile Mezereum,
   Verdünnungsgrad D2 bis D4;
   (d) 0,5 bis 2 Gew.-Teile Ranunculus,
   Verdünnungsgrad D2 bis D4;
   (e) 0,5 bis 2 Gew.-Teile Veratrum album,
   Verdünnungsgrad D3 bis D6;
   (f) 0,5 bis 2 Gew.-Teile Aconitum,
   Verdünnungsgrad D3 bis D6; und
   (g) 0,5 bis 2 Gew.-Teile Arnica,
   Verdünnungsgrad D2 bis D4.

5. Das Warzenmittel nach Anspruch 1 oder 2,
   gekennzeichnet durch
   ein peroral verabreichbares Präparat, enthaltend
   (a) 8,3 9 Euphorbia cyparissias,
   Verdünnungsgrad D3 bis D6;
   (b) 8,3 9 Clematis,
   Verdünnungsgrad D2 bis D4;
   (c) 8,3 9 Mezereum,
   Verdünnungsgrad D2 bis D4;
   (d) 8,3 9 Ranunculus,
   Verdünnungsgrad D2 bis D4;
   (e) 8,1 9 Veratrum album,
   Verdünnungsgrad D3 bis D6;
   (f) 7,2 9 Aconitum,
   Verdünnungsgrad D3 bis D6;
   (g) 7,2 9 Arnica,
   Verdünnungsgrad D2 bis D4; und
   (h) 8,3 9 Sulfur,
   Verdünnungsgrad D4 bis D6.

6. Das Warzenmittel nach Anspruch 1 oder 2,
   dadurch gekennzeichnet, daß
   das Gemisch zusätzlich
   (h) 0,5 bis 2 Gew.-Teile Atropa belladonna,
   Verdünnungsgrad D3 bis D6
   und/oder zusätzlich
   (i) 0,5 bis 2 Gew.-Teile Cinchona succirubra (China),
   Verdünnungsgrad D2 bis D4
   und/oder zusätzlich
   (k) 0,5 bis 2 Gew.-Teile Eucalyptus globulus,
   Verdünnungsgrad D2 bis D4
   und/oder zusätzlich
   (l) 0,5 bis 2 Gew.-Teile Lycopodium,
   Verdünnungsgrad D3 bis D6
   und/oder zusätzlich
   (m) 0,5 bis 2 Gew.-Teile Nux vomica,
   Verdünnungsgrad D3 bis D6
   enthält.

7. Das Warzenmittel nach Anspruch 6,
   gekennzeichnet durch
   ein peroral verabreichbares Präparat, enthaltend

(a) 8,3 9 Euphorbia cyparissias,
Verdünnungsgrad D3 bis D6;
(b) 8,3 9 Clematis,
Verdünnungsgrad D2 bis D4;
(c) 8,3 9 Mezereum,
Verdünnungsgrad D2 bis D4;
(d) 8,3 9 Ranunculus,
Verdünnungsgrad D2 bis D4;
(e) 8,1 9 Veratrum album,
Verdünnungsgrad D3 bis D6;
(f) 7,2 9 Aconitum,
Verdünnungsgrad D3 bis D6;
(g) 7,2 9 Arnica,
Verdünnungsgrad D2 bis D4;
(h) 7,2 9 Atropa belladonna,
Verdünnungsgrad D3 bis D6;
(i) 7,2 9 Cinchona succirubra (China),
Verdünnungsgrad D2 bis D4;
(k) 7,2 9 Eucalyptus globulus,
Verdünnungsgrad D2 bis D4;
(l) 7,2 9 Lycopodium,
Verdünnungsgrad D3 bis D6;
(m) 7,2 9 Nux vomica,
Verdünnungsgrad D3 bis D6; und
(n) 8,3 9 Sulfur,
Verdünnungsgrad D4 bis D6.

8. Verwendung wenigstens einer der in den Ansprüchen 1 bis 7 angegebenen Zusammensetzungen in den dort angegebenen Verdünnungen zur Herstellung eines peroral verabreichbaren Arzneimittels zur Behandlung von durch Papillomviren hervorgerufener Warzen an Mensch und Tier.

**Claims**

1. A pharmaceutic c preparation n for the treatment of warts,
comprising components of the plant Euphorbia cyparissias,
characterized by
a perorally administrable mixture of original tinctures comprising a content of
a) 0.5 to 2 parts by weight of Euphorbia cyparissias extract,
degree of dilution D3 to D6;
b) 0.5 to 2 parts by weight of Clematis recta extract,
degree of dilution D2 to D4; and
c) 0.5 to 2 parts by weight of Mezereum extract,
degree of dilution D2 to D4.

2. The pharmaceutic preparation for the treatment of warts according to claim 1,
characterized in that

said mixture contains additionally an original tincture of sulfur,
degree of dilution D2 to D6.

3. The pharmaceutic preparation for the treatment of warts according to claim 1 or 2,
characterized in that
said mixture contains additionally
d) 0.5 to 2 parts by weight of Ranunculus extract,
degree of dilution D2 to D4
and/or contains additionally
e) 0.5 to 2 parts by weight of Veratrum album extract,
degree of dilution D3 to D6,
and/or contains additionally
f) 0.5 to 2 parts by weight of Aconitum extract,
degree of dilution D3 to D6,
and/or contains additionally
g) 0.5 to 2 parts by weight of Arnica extract,
degree of dilution D2 to D4.

4. The pharmaceutic preparation for the treatment of warts according to claim 1 or 2,
characterized by
a perorally administrable preparation comprising
a) 0.5 to 2 parts by weight of Euphorbia cyparissias extract,
degree of dilution D3 to D6;
b) 0.5 to 2 parts by weight of Clematis extract,
degree of dilution D2 to D4;
c) 0.5 to 2 parts by weight of Mezereum extract,
degree of dilution D2 to D4;
d) 0.5 to 2 parts by weight of Ranunculus extract,
degree of dilution D2 to D4;
e) 0.5 to 2 parts by weight of Veratrum album extract,
degree of dilution D3 to D6;
f) 0.5 to 2 parts by weight of Aconitum extract,
degree of dilution D3 to D6; and
g) 0.5 to 2 parts by weight of Arnica extract,
degree of dilution D2 to D4.

5. The pharmaceutic preparation for the treatment of warts according to claim 1 or 2,
characterized by
a perorally administrable preparation comprising
a) 8.3 g of Euphorbia cyparissias extract,
degree of dilution D3 to D6;
b) 8.3 g of Clematis extract,
degree of dilution D2 to D4;

c) 8.3 g of Mezereum extract,
degree of dilution D2 to D4;
d) 8.3 g of Ranunculus extract,
degree of dilution D2 to D4;
e) 8.1 g of Veratrum album extract,
degree of dilution D3 to D6;
f) 7.2 g cf Aconitum extract,
degree of dilution D3 to D6;
g) 7.2 g of Arnica extract,
degree of dilution D2 to D4; and
h) 8.3 g of sulfur extract,
degree of dilution D4 to D6.

6. The pharmaceutic preparation for treatment of warts according to claim 1 or 2,
characterized in that
said mixture contains additionally
h) 0.5 to 2 parts by weight of Atropa belladonna extract,
degree of dilution D3 to D6.
and/or contains additionally
i) 0.5 to 2 parts by weight of Cinchona succirubra (China) extract,
degree of dilution D2 to D4;
and/or contains additionally
k) 0.5 to 2 parts by weight of Eucalyptus globulus extract,
degree of dilution D2 to D4;
and/or contains additionally
l) 0.5 to 2 parts by weight of Lycopodium extract,
degree of dilution D3 to D6;
and/or contains additionally
m) 0.5 to 2 parts by weight of Nux vomica extract,
degree of dilution D3 to D6.

7. The pharmaceutic preparation for the treatment of warts according to claim 6,
characterized by
a perorally administrable preparation comprising
a) 8.3 g of Euphorbia cyparissias extract,
degree of dilution D3 to D6;
b) 8.3 g of Clematis extract,
degree of dilution D2 to D4;
c) 8.3 g of Mezereum extract,
degree of dilution D2 to D4;
d) 8.3 g of Ranunculus extract,
degree of dilution D2 to D4;
e) 8.1 g of Veratrum album extract,
degree of dilution D3 to D6;
f) 7.2 g of Aconitum extract,
degree of dilution D3 to D6;
g) 7.2 g of Arnica extract,
degree of dilution D2 to D4;
h) 7.2 g of Atropa belladonna extract,
degree of dilution D3 to D6;

i) 7.2 g of Cinchona succirubra (China) extract,
degree of dilution D2 to D4;
k) 7.2 g of Eucalyptus globulus extract,
degree of dilution D2 to D4;
l) 7.2 g of Lycopodium extract,
degree of dilution D3 to D6;
m) 7.2 g of Nux vomica extract,
degree of dilution D3 to D6; and
n) 8.3 g of sulfur extract,
degree of dilution D4 to D6.

8. Use of at least one preparation having a composition and a degree of dilution as stated in anyone of claims 1 to 7
for preparing a perorally administrable pharmaceutic preparation for the treatment of warts caused by papillomaviruses in humans and animals.

## Revendications

1. Une préparation pharmaceutique pour le traitement de verrues,
sur la base de composants de la plante Euphorbia cyparissias,
caractérisée par
un mélange administré de manière perorale de teintures originales ayant une teneur de
a) 0,5 à 2 parts en poids d'extrait d'Euphorbia cyparissias,
degré de dilution D3 à D6;
b) 0,5 à 2 parts en poids d'extrait de Clematis recta,
degré de dilution D2 à D4; et
c) 0,5 à 2 parts en poids d'extrait de Mezereum,
degré de dilution D2 à D4.

2. La préparation pharmaceutique pour le traitement de verrues suivant la revendication 1,
caractérisée en ce que
le mélange contient en outre une teinture originale de soufre,
degré de dilution D2 à D6.

3. La préparation pharmaceutique pour le traitement de verrues suivant la revendication 1,
caractérisée en ce que
le mélange contient en outre
d) 0,5 à 2 parts en poids d'extrait de Ranunculus,
degré de dilution D2 à D4;
et/ou contient en outre
e) 0,5 à 2 parts en poids d'extrait de Veratrum album,
degré de dilution D3 à D6;
et/ou contient en outre

f) 0,5 à 2 parts en poids d'extrait d'Aconitum,
degré de dilution D3 à D6
et/ou contient en outre

    g) 0,5 à 2 parts en poids d'extrait d'Arnica,
    degré de dilution D2 à D4.

4. La préparation pharmaceutique pour le traitement de verrues suivant la revendication 1 ou 2,
caractérisée par
une préparation administrée de manière perorale contenant

    a) 0,5 à 2 parts en poids d'extrait d'Euphorbia cyparissias,
    degré de dilution D3 à D6;

    b) 0,5 à 2 parts en poids d'extrait de Clematis recta,
    degré de dilution D2 à D4;

    c) 0,5 à 2 parts en poids d'extrait de Mezereum,
    degré de dilution D2 à D4;

    d) 0,5 à 2 parts en poids d'extrait de Ranunculus,
    degré de dilution D2 à D4;

    e) 0,5 à 2 parts en poids d'extrait de Veratrum album,
    degré de dilution D3 à D6;

    f) 0,5 à 2 parts en poids d'extrait d'Aconitum,
    degré de dilution D3 à D6; et

    g) 0,5 à 2 parts en poids d'extrait d'Arnica,
    degré de dilution D2 à D4.

5. La préparation pharmaceutique pour le traitement de verrues suivant la revendication 1 ou 2,
caractérisée par
une préparation administrée de manière perorale contenant

    a) 8,3 g d'extrait d'Euphorbia cyparissias,
    degré de dilution D3 à D6;

    b) 8.3 g d'extrait de Clematis recta,
    degré de dilution D2 à D4;

    c) 8,3 g d'extrait de Mezereum,
    degré de dilution D2 à D4;

    d) 8,3 g d'extrait de Ranunculus,
    degré de dilution D2 à D4;

    e) 8,1 g d'extrait de Veratrum album,
    degré de dilution D3 à D6;

    f) 7,2 g d'extrait d'Aconitum,
    degré de dilution D3 à D6;

    g) 7,2 g d'extrait d'Arnica,
    degré de dilution D2 à D4; et

    h) 8,3 g d'extrait de soufre,
    degré de dilution D4 à D6.

6. La préparation pharmaceutique pour le traitement de verrues suivant la revendication 1 ou 2,
caractérisée en ce que
le mélange contient en outre

    h) 0,5 à 2 parts en poids d'extrait d'Atropa belladonna,
    degré de dilution D3 à D6
et/ou contient en outre

    i) 0,5 à 2 parts en poids d'extrait de Cinchona succirubra (Chine),
    degré de dilution D2 à D4
et/ou contient en outre

    k) 0,5 à 2 parts en poids d'extrait d'Eucalyptus globulus,
    degré de dilution D2 à D4
et/ou contient en outre

    l) 0,5 à 2 parts en poids d'extrait de Lycopodium,
    degré de dilution D3 à D6
et/ou contient en outre

    m) 0,5 à 2 parts en poids d'extrait de Nux vomica,
    degré de dilution D3 à D6.

7. La préparation pharmaceutique pour le traitement de verrues suivant la revendication 6,
caractérisée par
une préparation administrée de manière perorale contenant

    a) 8,3 g d'extrait d'Euphorbia cyparissias,
    degré de dilution D3 à D6;

    b) 8,3 g d'extrait de Clematis recta,
    degré de dilution D2 à D4;

    c) 8,3 g d'extrait de Mezereum,
    degré de dilution D2 à D4;

    d) 8,3 g d'extrait de Ranunculus,
    degré de dilution D2 à D4;

    e) 8,1 g d'extrait de Veratrum album,
    degré de dilution D3 à D6;

    f) 7,2 g d'extrait d'Aconitum,
    degré de dilution D3 à D6;

    g) 7,2 g d'extrait d'Arnica,
    degré de dilution D2 à D4;

    h) 7,2 g d'extrait d'Atropa belladonna,
    degré de dilution D3 à D6;

    i) 7,2 g d'extrait de Cinchona succirubra (Chine),
    degré de dilution D2 à D4;

    k) 7,2 g d'extrait d'Eucalyptus globulus,
    degré de dilution D2 à D4;

    l) 7,2 g d'extrait de Lycopodium,
    degré de dilution D3 à D6;

    m) 7,2 g d'extrait de Nux vomica,
    degré de dilution D3 à D6; et

    n) 8,3 g d'extrait de soufre,
    degré de dilution D4 à D6.

8. Emploi d'au moins une préparation ayant la composition et le degré de dilution mentionnés respectivement aux revendications 1 à 7 pour la préparation d'un médicament administré de manière perorale pour le traitement de virrues d'hommes et d'animaux causées par des virus du papillome.